# EUROPEAN PATENT SPECIFICATION

(11) **EP 4 415 722 B1**
(45) Date of publication and mention of the grant of the patent: **25.11.2026**
(21) Application number: 22803175.3
(22) Date of filing: 12.10.2022
(51) Int. Cl.: A61K 31/5377, A61K 9/00, A61K 47/10, A61P 29/00, A61P 29/02, A61P 31/04

(54) **LOW BACK PAIN TREATMENT**
BEHANDLUNG VON RÜCKENSCHMERZEN
TRAITEMENT DE LA LOMBALGIE

(30) Priority: 12.10.2021 US 202163254801 P
(43) Date of publication of application: 21.08.2024
(73) Proprietor: Persica Pharmaceuticals Ltd., Canterbury, Kent CT4 6HD (GB)
(72) Inventor: CZAPLEWSKI, Lloyd, Canterbury Kent CT4 6HD (GB); HAMLYN, Peter John, Canterbury Kent CT4 6HD (GB); MCHALE, Duncan, Canterbury Kent CT3 1TB (GB); CUMMINGS, Paul John, Haverhill Suffolk CB9 9PL (GB)
(74) Representative: Harris, Jennifer Lucy
(86) International application number: PCT/EP2022/078403
(87) International publication number: WO 2023/062080

(56) References cited:
- WO-A1-2019/097242

## Description

### Field of the Disclosure

The present disclosure relates to treatment to alleviate pain and disability in patients with low back pain (LBP) resulting from infection, particularly chronic low back pain (CLBP).

### Background of the Disclosure

Chronic Low back pain (CLBP) is common among the general population worldwide. A positive association between Modic changes and non-specific CLBP has been reported. Modic change Type I, and Modic change Types I +II are found in a higher proportion of patients with non-specific CLBP than in the general population. Modic changes, characterized by edema (or inflammation) in vertebrae, are caused by infection of the spinal discs. Several pathogens including anaerobic bacteria, e.g., *Cutibacterium acnes (C. acnes)* (previously known as *Propionibacterium acnes (P. acnes)),* can infect intervertebral discs and cause Modic changes and low back pain.

Antibiotic therapy may be effective in the treatment of CLBP associated with Modic changes. For example, oral administration of antibiotics such as amoxicillin-clavulanate has a clinically important and statistically significant (p< 0.001) improvement in all outcome measures in patients with chronic LBP. Although several non-surgical treatment approaches including intradiscal injections of medicines have demonstrated some short-term efficacy in reducing Modic changes and CLBP in non-replicated clinical studies, none of these approaches are successful and some even cause controversial results.

The present disclosure addresses these unmet issues by providing antibiotic compositions and formulations and spinal injection methods and dosing regimens for treatment of low back pain, especially chronic low back pain (CLBP).

### Summary of the Disclosure

The present disclosure provides linezolid compositions for use in methods for treatment of low back pain, specifically chronic low back pain in a patient.

In one aspect, provided herein is an injectable composition comprising linezolid form II and a thermosensitive hydrogel delivery carrier comprising poloxamer 407 and iohexol, for use in a method for treating low back pain, the method comprising administering a therapeutically effective amount of linezolid form II to a patient; wherein
the composition comprises about 2.5% to 20% of linezolid form II, about 17% to 30% iohexol and about 10.8% to 12.8% poloxamer 407 by weight of the composition;
the composition is administered to one or more intervertebral discs;
the patient receives two doses of the composition in each of the one or more intervertebral discs;
the first and second doses contain about 150 mg linezolid form II for each dose; and
the dosing interval between the first dose and second dose is from about 72 hours to about 132 hours.

In some embodiments, linezolid is pre-prepared as sterilized micronized powder in a dose unit and loaded to the poloxamer 407 based thermosensitive hydrogel delivery carrier prior to the administration, wherein the thermosensitive hydrogel delivery carrier is pre-prepared as a sterilized solution. As a non-limiting example, linezolid (Form II) is pre-prepared in a dose unit of about 250 mg and loaded to about 5 ml of the thermosensitive hydrogel delivery carrier prior to the administration, forming a linezolid composition suitable for spinal administration.

The linezolid composition may be delivered to the intervertebral discs using an introducer needle and an administration needle that is connected to a luer lock syringe. In some examples, the linezolid composition may be delivered to the intervertebral discs using an administration needle only.

In some embodiments, the patient with low back pain has vertebral body endplate bone oedema (Modic change), such as Modic change Type I, Type II or Type I+II.

### Brief description of the drawings

Figure 1 shows a representative standard curve for linezolid detection in human plasma.
Figure 2 shows a representative standard curve for poloxamer 407 in surrogate matrix.
Figure 3 shows plasma linezolid concentrations after administration of 3 mL of PP353 (150 mg linezolid) on a log₁₀ scale vs time in hours with lines for each of the three patients.

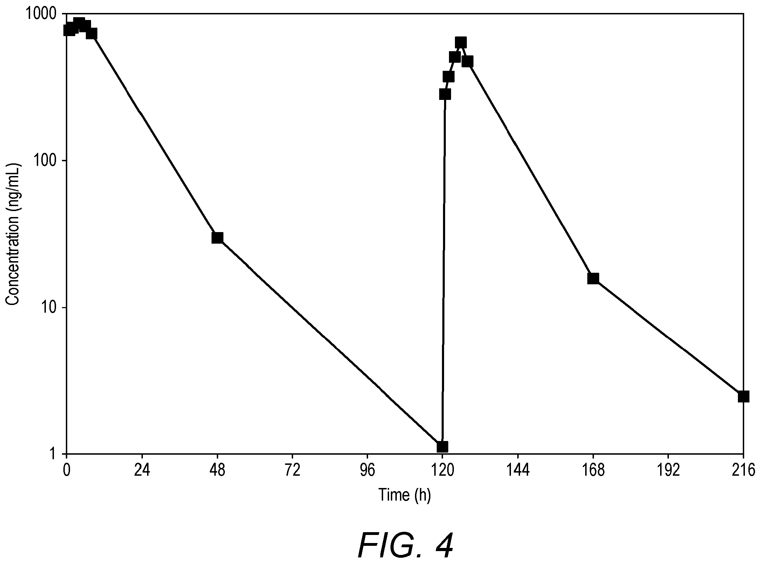

Figure 4 shows plasma linezolid concentration on a log₁₀ scale vs time in hours after administration of a 3 mL dose of PP353 (150 mg linezolid) to a patient, with a second 3mL dose administered 120 hours after the first dose.

### Detailed Description of the disclosure

The details of one or more embodiments of the disclosure are set forth in the accompanying description below. Unless otherwise defined, all technical and scientific terms used herein have the same meaning as commonly understood by one of ordinary skill in the art to which this disclosure belongs. The references to methods of treatment in the subsequent paragraphs of this description are to be interpreted as references to the compounds, pharmaceutical compositions and medicaments of the present invention for use in a method of treatment of the human body by therapy.

### I. Introduction

The present disclosure relates to specific treatment of low back pain such as CLBP, specifically low back pain associated with Modic changes. Low back pain may result from infection in the spine. The present disclosure provides antibiotic compositions and administration methods that are suitable for delivering an effective amount of medicine to the spine and/or spinal compartments.

The present disclosure discusses specific methods for preparing antibiotic compositions and dose units for effective delivery of antibiotics to targeted disease areas such as one or more spinal compartments. The antibiotic composition is formulated in a thermosensitive hydrogel composed of biodegradable poloxamer. The antibiotic composition is an aqueous suspension that is injectable. Upon administration to a patient, the solution gels at the targeted areas and slowly releases the antibiotic loaded in the hydrogel.

The present disclosure also discusses administration methods that can deliver an effective amount of antibiotic to the spine, for treating, preventing, ameliorating, and/or mitigating pain and/or adverse health consequences e.g. disability associated with pain. Particularly the present disclosure discusses injection methods for delivering a linezolid composition to one or more spinal sites for treating, preventing, ameliorating, and/or mitigating low back pain and disability and simultaneously eliminating bacterial infection in a cervical, thoracic, lumbar and/or sacral vertebra. The present treatment helps individuals suffering from acute or chronic low back pain to perform their daily living activities such as sitting, sleeping or driving without or with less pain. In particular, the present antibiotic compositions and administration methods can be used for treating chronic low back pain in a patient, e.g., low back pain that lasts 6 months or longer.

Compared to prolonged oral administration of antibiotics the present compositions and administration methods would reduce the level of systemic side effects, increase patient compliance to the dosing regime and increase efficacy at the site of action with a smaller antibiotic dosage. The advantages may include e.g., localized delivery for a site-specific action in the body, reduced dosing frequency without compromising the effectiveness of the treatment, reduction of side effects, faster onset of action and increased dosing compliance.

### II. Low back pain treatment

### Low back pain

Low back pain is common, ranging in intensity from a dull, constant ache to a sudden, sharp or shooting pain. It can be acute or chronic. Chronic low back pain (CLBP) is persistent, i.e., chronic, which may last for three months or longer. Types of pain may include, but are not limited to, acute pain, sub-acute pain, chronic or constant pain, local pain, radicular pain, referred pain, somatic pain, radiating pain, neuropathic pain, inflammatory pain, and mixed pain.

LBP can be disease specific or non-specific. As used herein, the term "non-specific low back pain" generally refers to low back pain for which the exact cause of the pain is unclear. It is defined as low back pain not attributable to a recognizable, known specific pathology (e.g., tumor, osteoporosis, lumbar spine fracture, structural deformity, inflammatory disorder, radicular syndrome, or cauda equina syndrome). Nonspecific low back pain is the most common type of back pain. The majority of cases of acute low back pain are classed as nonspecific. Nonspecific low back pain is classed as chronic if it lasts for longer than three months. In some people it lasts for months, or even years. As discussed herein, a subset of non-specific CLBP, i.e., CLBP with Modic changes, has been found to be associated with bacterial infection.

Low back pain substantially affects people's lifestyle, work and activities. The current management of low back pain offers low to moderate improvement in pain and disability. A variety of factors could cause low back pain. A strategy to identify subgroups of patients who share similar causes of low back pain would benefit to develop specific treatments.

It has been reported that patients with chronic low back pain of more than 6-months duration are often diagnosed with vertebral body endplate bone edema (Modic changes, MCs), such as Modic change type I, Modic change Type II, or Modic change type I + type II, at the painful disc level in the MRI.. Bacterial infection can induce a local inflammatory process that leads to vertebral end plate changes. Because Modic changes are strongly associated with LBP, the finding of the etiological correlation between Modic changes and bacterial infection provides a novel strategy for treating LBP, specifically CLBP. Antibiotics that can treat infection in the spine would treat low back pain associated with Modic changes resulting from infection.

### Antibiotic compositions

In accordance with the present disclosure, pharmaceutical compositions and formulations comprising antibiotics against bacterial infections that cause Modic changes and CLBP are used for treatment of low back pain, particularly CLBP.

In some examples disclosed herein, selection of antibiotics may depend on the bacterial pathogens isolated from Modic discs. The bacterial pathogens most frequently isolated from Modic discs are *Staphylococcus spp.* and C. *acnes.* Antibiotic resistances vary in different populations and territories worldwide. To have a robust and widely effective therapy, coverage of common resistances would be preferred with C. *acnes* and *Staphylococcus,* or with C. *acnes* only as a minimum. Preferably, antibiotics that are effective against current clinical isolates from any infection site may be selected as active agents of the present compositions and formulations, given the resistance profiles of pathogens isolated at the site of infection associated with Modic changes. In some examples disclosed herein, antibiotics that are effective against both C. *acnes* and *Staphylococci* may be selected as active agents of the present compositions and formulations. In some examples disclosed herein, a combination of the antibiotics that are effective against both C. *acnes* and *Staphylococci* may be selected. In some non-limiting examples, the antibiotic composition comprises at least one antibiotic for the treatment of the C. *acnes* infection that is associated with Modic change Type I.

According to the presently claimed invention, the antibiotic is linezolid (chemical name: ((S)-N-((3-(3-fluoro-4-morpholinophenyl)-2-oxooxazolidin-5-yl)methyl)acetamide) form II. Linezolid is an oxazolidinone against most Gram-positive bacteria, including *streptococci,* vancomycin-resistant *enterococci* (VRE), and methicillin-resistant *Staphylococcus aureus* (MRSA). Linezolid is well absorbed, with a bioavailability of approximately 100% in healthy volunteers. Linezolid can penetrate to tissues relatively quickly to reach its minimum inhibitory concentration (MIC) at 1 mg/L. In accordance, the linezolid compositions as discussed herein are formulated to deliver a therapeutically effective amount of linezolid to one or more target sites in a subject in need. The effective amount of linezolid is provided based, at least in part, on the target bacteria, means of administration, and other determinants. In general, an effective amount of linezolid provides efficient killing or inhibition of target bacteria and reduces pain or the risk of developing pain in the subject in need.

According to the presently claimed invention, linezolid form II as the active pharmaceutical ingredient (the API) is formulated in a delivery carrier. In examples disclosed herein, different polymorphic forms (i.e., crystal modifications) of linezolid can be selected as the active ingredient of the composition. For example, linezolid can be linezolid form I (e.g., U.S. Pat. No. 6,444,813), or form III (e.g., US. Pat. NO. 7,718,799; U.S. Patent publication No. 2007/0104785), or form IV (e.g., U.S. Patent Publication No. 2008/0319191), or other crystal forms as described in PCT Application Publication Nos. WO2007/026369, WO2006/110155 and WO2014/013498; and U.S. Patent Publication No. 2017/0008919. As detailed in U.S. Pat. No. 6,559,305, Linezolid ((S)-N-[[3-[3-fluoro-4-(4-morpholinyl)phenyl]-2-oxo-5-oxazolidinyl] methyl] acetamide) form II may be characterized by a powder X-ray diffraction (XRPD) spectrum having the following peaks:

| d-Spacing (Å) | Two-Theta Angle (°) | Relative Intensity (%) |
|---|---|---|
| 12.44 | 7.10 | 2 |
| 9.26 | 9.54 | 9 |
| 6.37 | 13.88 | 6 |
| 6.22 | 14.23 | 24 |
| 5.48 | 16.18 | 3 |
| 5.28 | 16.79 | 100 |
| 5.01 | 17.69 | 2 |
| 4.57 | 19.41 | 4 |
| 4.50 | 19.69 | 2 |
| 4.45 | 19.93 | 6 |
| 4.11 | 21.61 | 15 |
| 3.97 | 22.39 | 23 |
| 3.89 | 22.84 | 4 |
| 3.78 | 23.52 | 7 |
| 3.68 | 24.16 | 1 |
| 3.52 | 25.28 | 13 |
| 3.34 | 26.66 | 1 |
| 3.30 | 27.01 | 3 |
| 3.21 | 27.77 | 1 |

For example, linezolid form II may be characterised by an XPRD pattern comprising peaks at 16.8, 14.2 and 22.4 degrees two theta. The XPRD pattern may further comprise peaks at 21.6 and 25.3 degrees two theta. When a crystalline form is identified using one or more XRPD peaks given as angles 2θ (two theta), each of the 2θ values is understood to mean the given value ± 0.2 degrees, unless otherwise expressed.
As detailed in U.S. Pat. No. 6,559,305, Linezolid ((S)-N-[[3-[3-fluoro-4-(4-morpholinyl)phenyl]-2-oxo-5-oxazolidinyl] methyl] acetamide) form II may be further characterised by an infrared (IR) spectrum as a mineral oil mull having the following peaks: 3364, 1748, 1675, 1537, 1517, 1445, 1410, 1401, 1358, 1329, 1287, 1274, 1253, 1237, 1221, 1145, 1130, 1123, 1116, 1078, 1066, 1049, 907, 852, and 758 cm⁻¹.

As a non-limiting example, linezolid can be linezolid form II, in milled powder form. Linezolid (Form II) can be milled into micronized powder and sterilized for preparing sterile injectable compositions of the present disclosure, using any suitable method. Preferably, the linezolid Form II is sterilized by gamma irradiation. The linezolid Form II may be prepared and packed separately.

In some embodiments, linezolid is prepared as an aqueous suspension at room temperature, while after *in vivo* injection, the suspension can transit into a non-flowing/stiff gel at body temperature. Over several hours or days, the gels break down (i.e., biodegradable).

According to the presently claimed invention, the_thermosensitive hydrogel delivery carrier comprises poloxamer 407 and iohexol. In some examples disclosed herein, any suitable delivery carriers may be used for delivering antibiotics (e.g., linezolid) to the spine. The delivery carrier may be suitable for injection, particularly for injection into the spine or areas near to the spine. For example, the delivery carrier may be an aqueous solution, a low viscous solution, a suspension, or a reversible thermogel. The carrier preferably is a biodegradable and biocompatible carrier. As used herein, the term "biocompatible" means the carriers are not toxic to the tissues and cells. As used herein, the terms "biodegradable" and "bioabsorbable" are used interchangeably. The biodegradation or bioabsorbance refers to the degradation, disassembly, digestion or disappearance of the delivery materials after releasing formulated therapeutically antibiotics (e.g., linezolid), in the biological environment.

According to the presently claimed invention, the delivery carrier is a thermosensitive hydrogel. The injectable thermosensitive hydrogels may have a solution-gel transition temperature around or below physiological temperature.

According to the presently claimed invention, the injectable thermosensitive hydrogel is composed of poloxamer. The antibiotic- thermosensitive hydrogel compositions provide local delivery of an effective amount of linezolid to a diseased site/sites, such as one or more spinal compartments.

Poloxamers are FDA-approved thermosensitive synthetic polymers, which are nonionic triblock copolymers composed of a central hydrophobic chain of polyoxypropylene (poly(propylene oxide)) flanked by two hydrophilic chains of polyoxyethylene (poly(ethylene oxide)). Biocompatible poloxamers have been widely used for drug delivery and tissue engineering. Poloxamer-based hydrogels allow reversible gelation under certain physiological temperature and pH by adjusting the composition of PEO and PPO, and the overall molecular weight and concentration.

According to the presently claimed invention, the thermosensitive hydrogel is composed of poloxamer 407 (also known as Pluronic^{®} F-127, Kolliphor 407, and SynperonicPE/F 127). Poloxamer 407 is triblock copolymer consisting by weight of approximately 70% PEO (polyethylene glycol) and 30% PPO (polypropylene oxide) with an average molecular weight of 11500. Poloxamer 407 exhibits thermoreversible gelation behavior. At a concentration of pure 20% (w/w), Poloxamer 407 is liquid in an aqueous solution at or below room temperature (~25 °C), but forms a soft gel at body temperature.

According to the presently claimed invention, the poloxamer 407 based thermosensitive hydrogel further comprises the radiocontrast agent iohexol to facilitate the application of the linezolid formulation to a target site/sites, for example, an intervertebral disc. The addition of a radiocontrast agent in the present antibiotic formulations will assist a clinic practitioner (like a physician) to see the product being administered, and monitor the condition of the disc being administered using e.g., fluoroscopy or CT. This real-time information can help the practitioner to decide when to stop injection when the disc is full and is starting to leak.

According to the presently claimed invention, the ratio and concentrations of poloxamer 407 and iohexol in the delivery carrier are optimized in the context of linezolid concentrations and gelling temperature and/or other parameters. The amounts of iohexol and poloxamer 407 in the delivery carrier are optimized to achieve the target temperature range for the solution to gel transition of the present linezolid thermosensitive hydrogel formulations.

In one preferred embodiment, the poloxamer 407 and iohexol containing delivery carrier is prepared as a separate solution. As discussed above, the concentrations of poloxamer and iohexol are optimized to certain concentrations so that the gelation temperature of the solution is optimized at or close to the body temperature. As a non-limiting example, the concentrations of iohexol and poloxamer 407 are optimized to achieve a gelling temperature ranging from 26°C to 38°C, or from 26°C to 32°C, or from 32°C to 36°C.

In one preferred embodiment, the delivery carrier comprises poloxamer 407 with about 10% to about 17% by weight of the delivery carrier, or at a concentration of about 121mg/ml to about 207mg/ml by volume of the carrier. Preferably it may comprise poloxamer 407 with about 11.5% to about 13.5% by weight of the carrier, or at a concentration of about 140 mg/ml to 165mg/ml in the carrier. In other embodiments, the carrier comprises iohexol with about 14.5% to about 62.5% by weight of the carrier, or at a concentration of about 174mg/ml to about 755mg/ml in the carrier. Preferably the delivery carrier may comprise iohexol with about 18 % to about 35% by weight of the carrier, or at a concentration of about 206 mg/ml to 425 mg/ml in the carrier. The delivery carrier as described herein can be used to deliver any drug, for example an antibiotic, for delivering to a deep cavity of the body.

The delivery solution is prepared at cold temperature and stored separately. As a non-limiting example, the poloxamer hydrogel solution may be made following the steps of (1) preparing a cold iohexol solution by adding iohexol to a solution comprising tromethamine and calcium disodium EDTA (pH at about 8.0); and (2) adding poloxamer 407 powder slowly to the cold iohexol solution and stirring the solution until the poloxamer powder is completely dissolved, wherein the poloxamer powder is added as portions. The poloxamer-iohexol solution may be sterilized and packed into separate vials, or syringes (e.g., dual chamber syringes) ready for use (e.g., PP353-B).

Linezolid compositions used for the present disclosure may further comprise one or more pharmaceutically-acceptable excipients, as suited to the particular dosage form desired. Various excipients for formulating pharmaceutical compositions and techniques for preparing the composition are known in the art (see Remington: The Science and Practice of Pharmacy, 21st Edition, A. R. Gennaro, Lippincott, Williams & Wilkins, Baltimore, MD, 2006). The use of a conventional excipient medium may be contemplated within the scope of the present disclosure, except insofar as any conventional excipient medium may be incompatible with a substance or its derivatives, such as by producing any undesirable biological effect or otherwise interacting in a deleterious manner with any other component(s) of the pharmaceutical composition.

Prior to use, a linezolid preparation such as a unit of pre-prepared sterilized powder of linezolid Form II (e.g., PP353-A) is loaded to a delivery carrier (e.g., a thermosensitive hydrogel delivery carrier, e.g., PP353-B) to form the linezolid composition (i.e., linezolid suspension). The linezolid composition may comprise a thermosensitive poloxamer hydrogel loaded with an effective amount of linezolid, a non-ionic contrast agent iohexol at a concentration which is optimized for the poloxamer solution to gel transition, and optionally one or more pharmaceutically acceptably excipient. The linezolid Form II, which is milled to form micronized powder and sterilized by gamma irradiation, forms a suspension in the poloxamer-iohexol thermosensitive hydrogel carrier. The linezolid Form II micronized powder may have a D90 of about 50 µm or less, optionally about 20 µm or less, optionally about 10 µm or less, optionally from about 3 to about 10 µm. The linezolid Form II micronized powder may have a D10 of about 5 µm or less, optionally about 3 µm or less, optionally about 1 µm or less, optionally from about 0.1 to about 1 µm. As used herein, the terms D10 and D90 have their conventional meaning as known to the person skilled in the art and can be measured by art-known particle size measuring techniques such as, for example, static light scattering, sedimentation field flow fractionation, photon correlation spectroscopy, laser diffraction or disk centrifugation. Preferably, D10 and D90 are measured using laser diffraction.

Linezolid may be loaded to the delivery carrier at a concentration ranging from about 2.5% to about 20% or about 2.5% to about 10%, or about 3.0% to about 10% by weight or by volume of the composition. In one aspect, the linezolid formulation may comprise about 2.5%, 5%, 7.5%, 10%, 12.5%, 15%, 17.5% or 20% linezolid by weight of the final composition. Linezolid may be present at a concentration from about 10mg/ml to about 200mg/ml, or from about 20mg/ml to about 200mg/ml, or from about 50mg/ml to about 200mg/ml. Particularly linezolid may be present in the formulation at a concentration of 10mg/ml, 25mg/ml, 30mg/ml, 35mg/ml, 40mg/ml, 45mg/ml, 50mg/ml, 55mg/ml, 60mg/ml, 65mg/ml, 70mg/ml, 75mg/ml, 80mg/ml, 85mg/ml, 90mg/ml, 100mg/ml, 150mg/ml, or 200mg/ml.

In one example disclosed herein, the linezolid composition comprises (i) about 1% to about 20% linezolid by weight of the formulation (w/w); (ii) about 9.5% to about 14.5 % poloxamer 407 by weight of the formulation (w/w) (i.e., at a concentration of about 115mg/ml to about 173mg/ml in the composition), or about 10.8% to about 12.8% poloxamer 407 by weight of the formulation (i.e., at a concentration of about 130mg/ml to about 156mg/ml in the composition); and (iii) about 14% to 59% iohexol by weight of the formulation (w/w) (i.e., at a concentration of about 165mg/ml to about 718mg/ml in the formulation) or about 17% to about 30% iohexol by weight of the formulation (i.e., at a concentration of about 206mg/ml to 364mg/ml in the formulation). As a non-limiting example, the linezolid formulation comprises about 5% w/w linezolid, about 11.8% w/w poloxamer 407 and about 27.2% w/w iohexol. In some embodiments, the aqueous linezolid composition may gel at about 26 °C, or about 27 °C, or about 28 °C, or about 30 °C, or about 31 °C, or about 32 °C, or about 33 °C, or about 34 °C, or about 35 °C, or about 36 °C, or about 37 °C, or about 38 °C. In one non-limiting example, the linezolid composition gels at about 28 °C. Linezolid can diffuse from the stiff gel. Over several days the gel breaks down.

According to the presently claimed invention, the linezolid composition for spinal administration comprises about 2.5 to 20% linezolid form II, about 17% to 30% iohexol, and about 10.8% to 12.8% poloxamer by weight of the composition (e.g., PP353).

The linezolid homogenous suspension (e.g., PP353) mixed prior to the application may be taken up into an injection device (e.g., a dual chambered syringe) and prepared with the intended dose volume. In some embodiments, the linezolid composition is administered using a dual chambered syringe. The linezolid composition is sterilized using any suitable methods.

The linezolid composition is injectable. The injectable antibiotic composition is formulated for delivering an effective amount of the antibiotic to the spine of a subject, including but not limited to, an intervertebral disc, intervertebral space, intra-articular space , ligamentum flavum, ligament associated with the spine (e.g., interspinous ligament and anterior/posterior longitudinal ligament), epidural space, tendon associated with the spine, site adjacent to bone edema, bone junction, facet joint, and/or other spinal compartments.

### Dosage and dosing regimens

In accordance, antibiotic compositions and methods for spinal administration as discussed in the present disclosure ensure delivery of an effective amount of antibiotic to the intervertebral discs for treating, ameliorating, and/or mitigating low back pain and/or phenotypic presentation, e.g., disability, found to be coincident with diseases, conditions or disorders associated with Modic changes or bone edema caused by bacteria infection.

In some examples disclosed herein, a single dose or multiple doses of the antibiotic composition may be administered to a patient for chronic low back pain treatment. Reference to a single dose or multiple doses may refer to a single dose or multiple doses in each of the one or more targeted site in the spine, such as in each of the one or more intervertebral discs.

In some examples disclosed herein, a single dose of the linezolid composition is administered for treating CLBP. The single dosage brings an effective amount of linezolid to inhibit bacterial infection. In accordance, a dosage of linezolid provides an effective amount of linezolid above the minimum inhibitory concentration (MIC) of the target bacteria. The target bacteria are Gram-positive bacteria, such as C. *acnes, Corynebacterium propinquum,* or those of the genus *Staphylococcus.* The MIC may range from 0.1-16mg/L. In a non-limiting example, a single dose of the linezolid suspension (e.g., PP353) may deliver about 10mg to about 200mg linezolid, or 10mg to 100mg, or 10mg to 150mg, or 20mg to 100mg, or 20mg to 150mg, or 20mg to 200mg, or 30mg to 150 mg, or 30 mg to 200mg, or 50 mg to 100 mg, or 50 mg to 150 mg, or 50mg to 200mg, or 100 mg to 120 mg, or 100mg to 150 mg, or 120 mg to 200mg linezolid. According to the presently claimed invention, a dose of the linezolid composition contains 150 mg linezolid.

According to the present invention, the patient receives two doses of the composition in each of the one or more intervertebral discs and the dosing interval between the first dose and second dose is from about 72 hours to about 132 hours. In some examples disclosed herein, a dosing interval (e.g., time between doses during a dosing regimen) is, about 24 hours, 48 hours, 72 hours, 96 hours, 120 hours, 144 hours, 168 hours, or longer. In some examples disclosed herein, a dosing interval is from about 24 hours to about 168 hours, from about 48 hours to about 144 hours, or from about 72 hours to about 120 hours. In some examples, a dosing interval is from about 72 hours to about 168 hours, from about 72 hours to about 144 hours, from about 72 hours to about 132 hours, or from about 72 hours to about 120 hours. In some embodiments, the patient is administered a defined number of doses of the linezolid composition in a defined period of time. For example, the patient may receive 2 doses of the linezolid composition over 72 hours, 96 hours, or 120 hours. The dosing regimens including specific amount, administration number, and frequency of doses over a specific period of time, are determined following clinical observations.

In some embodiments, a single dosing interval is used over the course of pain treatment with the linezolid composition. In some embodiments, multiple dosing intervals are used over the course of pain treatment with the linezolid composition. In some embodiments, a dosing interval is fixed. In some embodiments, a dosing interval is an individualized interval that is determined for a given subject based on, e.g., pharmacokinetic/toxicokinetic (PK/TK) data or other information about that subject, such as age, weight, height, body mass index, disease severity, comorbidities, race, ethnicity, national origin, genotype, and/or prior response to the antibiotic treatment. An individualized dose and dosing interval combination may be the same as those for fixed interval regimens or may differ.

In some embodiments, a dosing regimen (e.g., the first dosing regimen and/or the second dosing regimen) including dose frequency, dose and duration time may be personalized according to the subject's reaction, side effects, and treatment efficacy.

### Spinal administration

In order to treat low back pain, antibiotics need to be delivered close to the infected sites. Any administration routes which result in a therapeutically effective outcome can be used. In one preferred embodiment, injection, particularly spinal injection may be used, to produce an effective level of linezolid (above MIC of target bacteria) to bring beneficial outcomes (e.g., pain relief and reduced disability).

In some examples disclosed herein, the antibiotic composition may be administered to vertebrae, intervertebral disc, intervertebral space, intra-articular space, ligamentum flavum, ligament associated with the spine (e.g., interspinous ligament, and anterior/posterior longitudinal ligament), epidural space, tendon associated with the spine, site adjacent to bone edema, bone junction, facet joint, and/or other spinal compartments.

In some examples disclosed herein, the antibiotic composition is administered to one or more vertebrae, intervertebral discs, intervertebral spaces, intra-articular spaces, ligamentum flavum, ligament associated with the spine, epidural space, tendon associated with the spine, site adjacent to bone oedema, bone junction, facet joint, and/or other spinal compartments.

Method and devices known in the art for spinal administration are contemplated for use in conjunction with the methods and compositions disclosed herein. These include, for example, those methods and devices having multiple needles, hybrid devices employing for example lumens or catheters as well as devices utilizing heat, electric current or radiation driven mechanisms.

In some embodiments, the antibiotic composition is injected to one or more intervertebral discs. A double needle system including an introducer needle and an administration needle may be used for intervertebral disc administration. An introducer needle may be used to for safely achieving access to the intervertebral space, which may have a beveled side opening at its distal tip which provides a tissue piercing point at the distal tip. The introducer needle may be positioned against the outer annulus fibrosis of the disc, or as close to the edge of outer annulus as possible. An administration needle is then used to inject an effective amount of the linezolid composition (e.g., PP353) directly in the center of the nucleus pulposus. In addition to aiding in the targeting of the disc, use of the introducer needle can avoid contact between the administration needle and the skin, thereby keeping the linezolid composition as cool as possible during administration and minimize risk of infection. The position of the introducer needle at the entry point is close to the foramen and at such an angle as to allow the tip of the administration needle to cross the disc to the center without penetrating the endplate. Under image guidance, the administration needle is advanced to the center of the nucleus pulposus across the line of diameter but not penetrate the far side of the annulus fibrosus. This guided injection can fill the disc pulposus with the linezolid composition (e.g., PP353), including as many of the degenerate splits in the annulus as may have occurred. This injection method brings a comprehensive fill but not one associated with material extravasation into adjacent spaces beyond the intervertebral space itself.

A dose of linezolid is administered to the intervertebral disc may be injected slowly over minutes, e.g., 1-10 minutes, or 1-5 minutes, or 2-10 minutes, or 2-8 minutes, or 3-10 minutes.

The present injection procedure provides several advantages over other methods for delivering antibiotics to the deep spine compartments. A major advantage is that the deep spinal compartments, such as intervertebral disc, intervertebral space, intra-articular space, ligamentum flavum, ligament (such as interspinous ligament and anterior/posterior longitudinal ligament), epidural space, tendon associated with the spine, site adjacent to bone edema, bone junction, facet joint, and/or other spinal compartments, are specifically targeted, allowing that the effective amount of antibiotic is locally delivered to the targeted area.

### III. Definitions

*Administer:* As used herein, the term "administer" or "administering" are used interchangeably herein to mean the delivery of a composition such as, for example, an antibiotic to a subject. Administration to a subject can include all suitable modes known to those of ordinary skill in the art. For example, administration as used in the present methods can include systemic or targeted modes of administration, such as injection, oral administration, topical administration, spinal injection, or intrathecal administration.

*Active pharmaceutical ingredient (API):* As used herein, the term "active pharmaceutical ingredient (API)" refers to a pharmaceutical agent that is biologically active. For example, a substance that when is administered to an organism, has a biological effect on that organism, is considered to be biologically active. In accordance with the present invention, the API is linezolid.

*Delivery carrier:* As used herein, the term "delivery carrier" refers to any agent, compound, or any combination thereof that can be used to carry an active ingredient (e.g., the API of the present invention) and deliver the same to a designated site. A delivery carrier can be, but is not limited to, a solution, a suspension, a hydrogel, a nanoparticle, a ligand and a virus.

*Dosing interval:* As used herein, the term "dosing interval" refers to the amount of time that elapses between multiple doses being administered to a subject.

*Formulation:* As used herein, a "formulation" includes at least an active ingredient and a delivery agent.

*Hydrogel:* As used herein, the term "hydrogels" are viewed as water insoluble, crosslinked, three-dimensional networks of polymer chains plus water that fills the voids between polymer chains. Crosslinking facilitates insolubility in water and provides required mechanical strength and physical integrity. Hydrogel is mostly water (the mass fraction of water is much greater than that of polymer). The ability of a hydrogel to hold significant amount of water implies that the polymer chains must have at least moderate hydrophilic character.

*MIC (minimum inhibitory concentration):* As used herein, the term "MIC" refers to the lowest concentration of an antimicrobial that will inhibit the visible growth of a microorganism after overnight incubation. A lower MIC value indicates that less drug is required for inhibiting growth of the organism; therefore, drugs with lower MIC scores are more effective antimicrobial agents.

*Patient:* As used herein, the term "patient" or "subject" refers to any organism who may seek or be in need of treatment, requires treatment, is receiving treatment, will receive treatment, or a subject who is under care by a trained professional for a particular disease or condition. The organism is a mammal such as mice, rats, rabbits, non-human primates, and humans. Preferably, a patient or subject is a human.

*Pharmaceutically acceptable:* As used herein, the phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication, commensurate with a reasonable benefit/risk ratio

*Pharmaceutical composition:* As used herein, the phrase "pharmaceutical composition" refers to a composition that alters the etiology of a disease, disorder and/or condition.

*Therapeutically effective amount:* As used herein, the term "therapeutically effective amount" means an amount of an agent to be delivered (e.g., antibiotic, drug, therapeutic agent, diagnostic agent, prophylactic agent, etc.) that is sufficient, when administered to a subject suffering from or susceptible to a disease, disorder, and/or condition, to treat, improve symptoms of, diagnose, prevent, and/or delay the onset of the disease, disorder, and/or condition.

*Treating:* As used herein, the term "treating" refers to partially or completely alleviating, ameliorating, improving, relieving, delaying onset of, inhibiting progression of, reducing severity of, and/or reducing incidence of one or more symptoms or features of a particular disease, disorder, and/or condition. Treatment may be administered to a subject who does not exhibit signs of a disease, disorder, and/or condition and/or to a subject who exhibits only early signs of a disease, disorder, and/or condition for the purpose of decreasing the risk of developing pathology associated with the disease, disorder, and/or condition.

### Equivalents and Scope

Those skilled in the art will recognize, or be able to ascertain using no more than routine experimentation, many equivalents to the specific embodiments in accordance with the invention described herein. The scope of the present invention is not intended to be limited to the above Description, but rather is as set forth in the appended claims.

In the claims, articles such as "a," "an," and "the" may mean one or more than one unless indicated to the contrary or otherwise evident from the context. Claims or descriptions that include "or" between one or more members of a group are considered satisfied if one, more than one, or all of the group members are present in, employed in, or otherwise relevant to a given product or process unless indicated to the contrary or otherwise evident from the context. The invention includes embodiments in which exactly one member of the group is present in, employed in, or otherwise relevant to a given product or process. The invention includes embodiments in which more than one, or the entire group members are present in, employed in, or otherwise relevant to a given product or process.

It is also noted that the term "comprising" is intended to be open and permits but does not require the inclusion of additional elements or steps. When the term "comprising" is used herein, the term "consisting of" is thus also encompassed and disclosed.

Where ranges are given, endpoints are included. Furthermore, it is to be understood that unless otherwise indicated or otherwise evident from the context and understanding of one of ordinary skill in the art, values that are expressed as ranges can assume any specific value or subrange within the stated ranges in different embodiments of the invention, to the tenth of the unit of the lower limit of the range, unless the context clearly dictates otherwise.

While the present invention has been described at some length and with some particularity with respect to the several described embodiments, it is not intended that it should be limited to any such particulars or embodiments or any particular embodiment, but it is to be construed with references to the appended claims so as to provide the broadest possible interpretation of such claims in view of the prior art and, therefore, to effectively encompass the intended scope of the invention.

### EXAMPLES

### Example 1: Pharmacokinetics (PK) of a single injection of PP353 in patients (reference example)

In this study, 3 patients were intradiscally injected with PP353; each patient received a single injection of 3ml (50mg/ml linezolid) of PP353.

### Preparation of PP353

PP353 is made up using a vial of PP353-A (253 mg micronized gamma-irradiated linezolid Form II powder) and PP353-B, a delivery solution for suspension of PP353-A which contains iohexol, poloxamer 407, other excipients and water for injection. PP353-A and PP353-B are kept cool *i.e.,* at 2-8°C prior to use.
PP353-A and PP353-B were prepared according to the procedures of Example 9 of WO 2019/097242.

As set out therein, specifications for the micronized Linezolid form II (PP353-A) were set at D10 0.2 to 1.0µm, D90 3 to 10µm, and the components of PP353-B were as follows:

| **PP353-B component** | **Quantity** |
|---|---|
| Poloxamer Kolliphor 407 | 149.50g |
| Iohexol | 342.65g |
| Calcium disodium EDTA | 0.100g |
| Tromethamine | 1.210g |
| 1M hydrochloric acid solution | As required |
| Water for Injection (WFI) | To 1196.0g |
| Intermediate formulation pH target (the pH value prior to Poloxamer addition and final formulation to target weight) | pH 8.00±0.20 |

The components of PP353-A and PP353-B, including linezolid Form II, were obtained commercially.

The preparation process for PP353 include the following steps:
(i) Invert the vial of PP353-A and gently tap on a hard surface 2-4 times to loosen any compacted powder from the base of the vial.
(ii) Remove the tear off portion of the aluminium seal and swab the top of the bung of the PP353-A vial with an alcohol impregnated lint free wipe.
(iii) Assemble a vent filter unit (MinisartTM PLUS 0.2µm pre-sterilized filter or equivalent and 19G 38mm (1.5" hypodermic needle (quincke point)); and Insert the vent filter unit into PP353-A positioning the needle tip below the vial shoulder and pointing towards the vial wall, without touching the powder base or the wall of the vial.
(iv) Select the vial of PP353-B and invert gently 5 times to mix, holding the vial by the aluminium seal and bottom edge of the vial ensuring minimal contact. Remove the plastic flip lid, and swab the bung of the PP353-B diluent for suspension with an alcohol impregnated lint free wipe.
(v) Using a 5 ml luer-lock syringe and 19 G 38 mm (1.5") quincke point needle (a sharp needle to minimize the risk of stopper coring) and good clinical injection practice, draw up approximately 5.3 ml PP353-B.
(vi) Replace the 19 G needle with a clean 19 G 38 mm (1.5") quincke point needle, expel any bubbles and prime the needle leaving a volume of 4.8 ml; Inject the 4.8 ml PP353-B into the PP353-A, by inserting the needle vertically in the center of the bung. The vial body can be held to ensure stability during this step.
(vii) Remove the needle and empty syringe and dispose safely and remove the vent and venting needle and dispose safely.
(viii) Gently shake the vial (now referred to as PP353) holding the aluminium seal and bottom edge of the vial between forefinger and thumb, ensuring minimal skin contact, for approximately 1-2 minutes to mix well until a visibly homogenous suspension is obtained; Inspect the base of the vial for solid powder.
(ix) Once the PP353 has reached a smooth milky consistency, with no obvious clumps of powder present, the preparation is complete. Kept at 2-8°C prior to use. PP353 may be used within 3 hours of preparation.

### Administration of PP353

PP353 is administered using a double needle technique. An introducer needle with an internal bore wide enough to insert the administration needle is positioned adjacent to the spinal disc to be injected after aseptic preparation and infiltration of skin with a suitable local anaesthetic agent. Preferably patients are conscious and alert during needle positioning so that they can report pain should the needle touch a nerve root.

Intradiscal injection of discs affected by Modic 1 endplate oedema may be painful. Appropriate anaesthesia may be used. Once paravertebral block/local anaesthesia is established, the administration needle is inserted into the introducer needle and advanced to the edge of the disc and then inserted to the center of the disc using image guidance. It is recommended that an 18G Quincke-point luer-lock spinal needle of at least 90 mm in length is used as an introducer (outer) needle and a 22G Quincke-point luer-lock spinal needle of up to 178 mm in length, is used as the administration (inner) needle.

### Dose Preparation

Dose preparation involves swabbing the bung of the PP353 vial (as discussed herein) with an alcohol impregnated lint free wipe. Using a 19G 38 mm (1.5") hypodermic needle (quincke-point), fill three sterile 1 ml luer-lock syringes with PP353 ensuring the absence of air bubbles and an accurate fill to the one ml marker. Place in the sterile area at 2-8°C. If air is noticed in the syringe subsequent to this preparation, the administrator, after excluding the air, can use their judgement to ensure that the subject is dosed correctly. Using a further 1 ml sterile syringe, prime the 10 cm luer-lock extension tubing with the PP353 preparation again ensuring all air bubbles are excluded and the fill is complete and replace the cap on the tubing. Connect the filled extension tube to one of the filled 1 ml syringes. The injection is performed as soon as possible to minimize warming of PP353 which would make it more viscous and therefore more difficult to inject. The administration needle should not be primed to avoid PP353 warming and becoming too viscous in the needle tract whilst the needle is being positioned.

### Needle Positioning

The introducer needle is positioned against the outer annulus of the disc, or as close to the edge of outer annulus as possible if not long enough. The purpose of the introducer needle is primarily to avoid contact between the intradiscal 22G needle and the skin to minimize risk of infection, to keep PP353 as cool as possible during administration, and to aid in the targeting of the disc. The entry point into the disc should be close to the foramen and at such an angle as to allow the tip of the intradiscal administration 22G needle to cross the disc to the center without penetrating the endplate. The introducer needle needs to be close to the ipsilateral foramen but not to impinge on the exiting nerve root which lies in the cranial part of the foramen.

A confirmatory AP and lateral screening/imaging is performed. If necessary, the needle is re-positioned until the appropriate positioning is achieved. Once positioned, the stylet is removed from the 18G needle.

Then the 22G administration needle is advanced to the center of the nucleus pulposus across the line of diameter under image guidance. The 22G needle needs not to penetrate the far side of the annulus fibrosus. The aim is to fill the disc pulposus with PP353 and for this "fill" to include as many of the degenerate splits in the annulus as may have occurred. This practice brings a comprehensive fill but not one associated with material extravasation into adjacent spaces beyond the intervertebral space itself.

### Dosing/Injection

After the needle is correctly positioned, the following steps are performed to finish the administration.
(i) Remove the stylet from the administration needle; and connect the primed connecting tubing and 1 ml syringe to the administration needle ensuring the luer-lock is secure and the introducer needle position is not altered;
(ii) Perform imaging at 90° to the trajectory of the needle to check the placement of the needle; and Inject 1 ml (one syringe full) of PP353 and perform imaging at 90° to the trajectory of the needle to check for extravasation;
(iii) Replace the empty 1 ml syringe with a second primed 1 ml syringe and inject a second 1 ml of PP353; and perform imaging at 90° to the trajectory of the needle to check for extravasation;
(iv) Replace the empty 1 ml syringe with the third and final full syringe and inject a third 1 ml of study medication; and perform imaging at 90° to the trajectory of the needle to check for extravasation; and
(v) Remove both the administration needle and the introducer needle together, and clean the puncture site and apply an occlusive sterile dressing.

### Pharmacokinetics

The pharmacokinetics of linezolid and poloxamer 407 were assessed in EDTA anticoagulated plasma samples at various times post intradiscal administration of a single injection of 3 ml of PP353. Plasma samples from injected patients are collected, for example, post-injection 30 minutes, 2 hours, 4 hours, 8 hours, 12 hours, 24 hours (day 2), 30 hours, 96 hours (day 5), 144 hours (day 7), 192 hours (day 9) and 240 hours (day 11). Plasma samples are also collected before injection (pre-dose).

No significant extravasation was reported in all three patients after a single dose of 3 ml of PP353.

### Linezolid assay

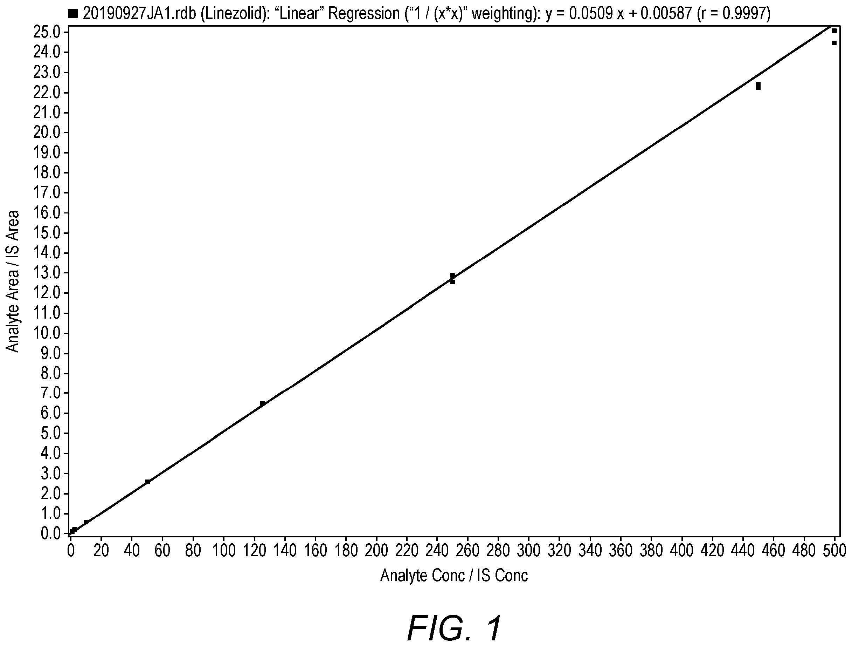

A high-performance liquid chromatography method (tandem mass spectrometric detection) is developed to estimate plasma linezolid with an AB Sciex API 4000 detector (ABS Laboratories, United Kingdom). Figure 1 shows representative standard curve for linezolid detection in human plasma.

### Poloxamer 407 assay

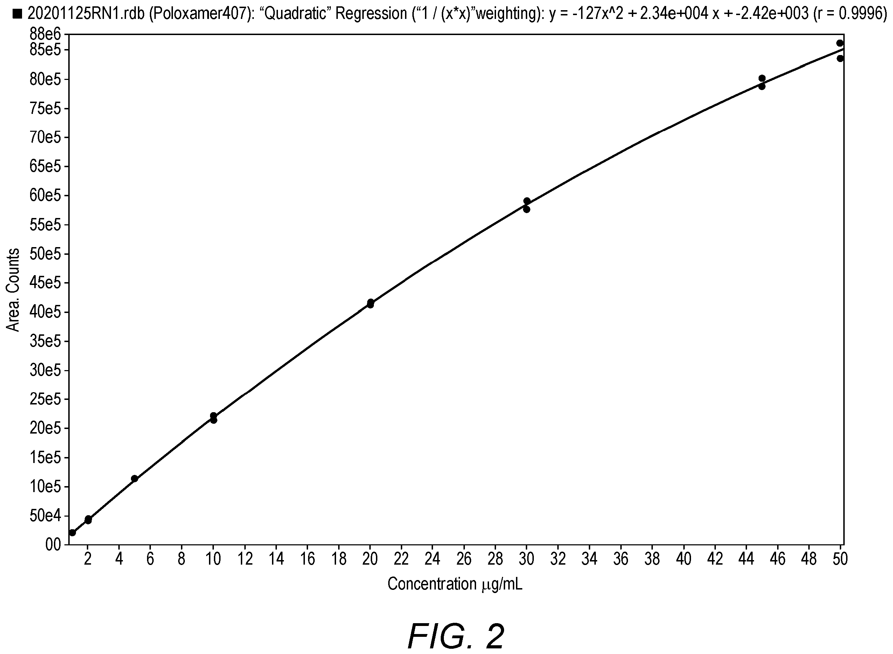

A high-performance liquid chromatography method (tandem mass spectrometric detection) is developed to estimate plasma Poloxamer 407 using an AB Sciex API 6500 detector (ABS Laboratories, United Kingdom). Figure 2 shows a representative standard curve for poloxamer 407 in surrogate matrix.

### Linezolid PKparameters for a 3mL intradiscal dose of PP353 in human

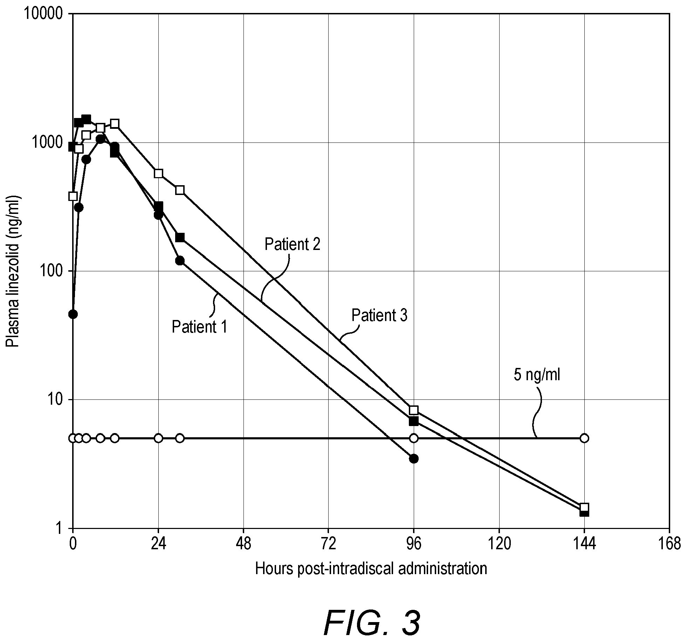

The maximum plasma linezolid concentration (Cₘₐₓ) was 1.0 to 1.5 µg/ml, while that observed for a standard 600 mg linezolid dose is approximately 15 µg/ml (Table 1: Figure 3). The Cₘₐₓ is about 10% of that observed for standard linezolid use. The monophasic elimination phase of the PK curves for three patients were parallel with a half-life of about 13 hours, as compared to the standard linezolid administration of 4-5 hours. The study used systemic plasma concentration as a surrogate for intradiscal linezolid concentration and prespecified 5 ng/ml as a plasma linezolid concentration which would indicate that the intradiscal depot was depleted. For this study that was 89 to 110 hours with a mean of 102 hours. The results indicate that the target of 8 days continuous intradiscal antibiotic exposure above the minimum inhibitory concentration (MIC) can be achieved by a second dose at approximately this time.

**Table 1. Linezolid PK parameters for a 3mL intradiscal dose of PP353 in human**

| **Patient** | **Cₘₐₓ** | **tₘₐₓ** | **AUC₀₋ₜ** | **λ_{z}** | **t_{½}** | **AUC_{0-∞}** | **CL/F** | **Tdur** |
|---|---|---|---|---|---|---|---|---|
| NO. | (ng/mL) | (h) | (h*ng/mL) | (1/h) | (h) | (h*ng/mL) | (mL/h) | (h) |
| 101-004 | 1060 | 8.00 | 18600 | 0.0576 | 12.0 | 18700 | 8040 | 89.4 |
| 101-006 | 1500 | 4.00 | 26300 | 0.0517 | 13.4 | 26300 | 5710 | 105 |
| 103-001 | 1390 | 12.0 | 34400 | 0.0511 | 13.6 | 34500 | 4350 | 110 |
| Arithmetic Mean | 1320 | 8.00 | 26400 | 0.0535 | 13.0 | 26500 | 6030 | 102 |
| Arithmetic SD | 229 | 4.00 | 7910 | 0.00355 | 0.832 | 7900 | 1860 | 10.8 |
| Minimum | 1060 | 4.00 | 18600 | 0.0511 | 12.0 | 18700 | 4350 | 89.4 |
| Median | 1390 | 8.00 | 26300 | 0.0517 | 13.4 | 26300 | 5710 | 105 |
| Maximum | 1500 | 12.0 | 34400 | 0.0576 | 13.6 | 34500 | 8040 | 110 |
| Geometric Mean | 1300 | 7.27 | 25600 | 0.0534 | 13.0 | 25700 | 5840 | 101 |
| Geometric CV% | 18.4 | 60.1 | 31.6 | 6.53 | 6.53 | 31.5 | 31.5 | 11.0 |

| | | | | | | | | |
|---|---|---|---|---|---|---|---|---|
| SD: Standard deviation; CV: Coefficient of variation; NC: Not calculated | | | | | | | | |

The target of 5 ng/ml of linezolid in plasma was set based on PK modelling. It is above the LLOQ for the assay of 1 ng/ml. Assessment of the Area Under the Curve (AUC) for the PK profiles for each patient allows an estimate of the linezolid eliminated from the disc at the time the 5 ng/ml plasma target was met and therefore an estimate of remaining linezolid may be made. The remaining linezolid was estimated to be approximately 200 µg per disc. This provides a suitable antibacterial exposure as a trough concentration prior to the second injection.

### Example 2: Pharmacokinetics (PK) after two doses of PP353 in patients

PP353 is prepared following the processes discussed in Example 1. Patients in this study are administered with two doses of PP353. The injection follows the same procedure as discussed in Example 1. PP353 is administered to the patient on Day 1 and then Day 5 (±1 day). Each injection includes 3ml of PP353 as discussed in Example 1.

About 40 patients are recruited to this clinical study, 20 patients are administered with PP353, the other half is on placebo. A dosing regimen that would provide approximately 8 days of continuous intradiscal linezolid exposure above an antibacterial concentration is targeted. This objective is achieved by two administrations of PP353 approximately 4 days apart on Day 1 and Day 5 (±1 day).

The pharmacokinetics of linezolid and poloxamer 407 are assessed in EDTA anticoagulated plasma samples at various times post each intradiscal administration of 3 ml of PP353. Plasma samples from injected patients are collected, for example, 30 minutes before first injection and after first dosing at, for example, 1 hour, 2 hours, 4 hours, 6 hours, 8 hours, 12 hours, 24 hours and 48 hours. On day 5 (±1 day), patients are administered to another dose of 3ml of PP353. On day 5 (±1 day), plasma samples are collected, for example, 30 minutes before second injection, and after second dosing at, for example, 1 hour, 2 hours, 4 hours, 6 hours, 8 hours. Samples are then collected after second dosing at, for example, 12 hours, 24 hours, 48 hours, 96 hours, and 144 hours. Plasma samples are also collected before injection (pre-dose).

### Interim results

One patient was injected with two intradiscal injections of PP353 of 3 mL on Days 1 and 6. Pharmacokinetic parameters of PP353 following these injections are presented in Table 2.

**Table 2. Pharmacokinetic parameters of PP353 following two intradiscal injections of PP353 of 3 mL**

| Dosing Day | Cₘₐₓ (ng/mL) | tₘₐₓ (h) | AUC₀₋ₜ (h*ng/mL) | AUC_{0-∞} (h*ng/mL) | λ_{z} **(1/h)** | t_{½} **(h)** | CL/F (mL/h) | Tdur (h) |
|---|---|---|---|---|---|---|---|---|
| 1 | 858 | 4 | 14900 | 15600 | 582 | 11.9 | 9650 | 87.1 |
| 6 | 644 | 6 | 9380 | NC | NC | NC | 15900 | 77.7 |

| | | | | | | | | |
|---|---|---|---|---|---|---|---|---|
| Tdur is the duration above 5 ng/mL | | | | | | | | |

Following single intradiscal injection of PP353 of 3 mL, maximum plasma concentrations (Cₘₐₓ) of 858 and 644 ng/mL were measured at 4 and 6 h, respectively post-dose. Plasma concentrations of PP353 declined approximately monophasically with an apparent terminal half-life of 12 h. The time that plasma concentrations of PP353 were above a prescribed limit of 5 ng/mL was 165 h (6.8 days) (Figure 3).
Plasma concentrations after the first dose appeared to be lower compared to subjects in Part A, presumably due to between-subject variability in systemic exposure to PP353. Plasma concentrations after the second dose appeared to be lower compared to the first dose, presumably due to within-subject variability in systemic exposure to PP353.

## Claims

1. An injectable composition comprising linezolid form II and a thermosensitive hydrogel delivery carrier comprising poloxamer 407 and iohexol, for use in a method for treating low back pain, the method comprising administering a therapeutically effective amount of linezolid form II to a patient; wherein
the composition comprises about 2.5% to 20% of linezolid form II, about 17% to 30% iohexol and about 10.8% to 12.8% poloxamer 407 by weight of the composition;
the composition is administered to one or more intervertebral discs;
the patient receives two doses of the composition in each of the one or more intervertebral discs;
the first and second doses contain about 150 mg linezolid form II for each dose; and
the dosing interval between the first dose and second dose is from about 72 hours to about 132 hours.

2. The composition for use of claim 1 wherein linezolid form II is formulated in a thermosensitive hydrogel delivery carrier composed of poloxamer 407 and iohexol; and wherein linezolid form II is pre-prepared as sterilized micronized powder in a dose unit and loaded to the poloxamer 407 based thermosensitive hydrogel delivery carrier prior to the administration, wherein the thermosensitive hydrogel delivery carrier is pre-prepared as a sterilized solution.

3. The composition for use of claim 2 wherein linezolid form II is pre-prepared in a dose unit of about 250 mg and loaded to about 5 ml of the thermosensitive hydrogel delivery carrier prior to the administration.

4. The composition for use of claim 2 or claim 3 wherein the composition is delivered to the one or more intervertebral discs using an introducer needle and an administration needle.

5. The composition for use of claim 2 or claim 3 wherein the composition is delivered to the one or more intervertebral discs using an administration needle.

6. The composition for use of claim 4 or claim 5 wherein the administration needle is connected to a luer-lock syringe.

7. The composition for use of claim 6 wherein the administration needle and the luer-lock syringe are connected via a flexible connector.

8. The composition for use of any one of claims 1 to 7 wherein the dosing interval between the first dose and second dose is from about 72 hours to about 120 hours.

9. The composition for use of claim 8 wherein the dosing interval between the first dose and second dose is 72 hours, 96 hours or 120 hours.

10. The composition for use of any preceding claim wherein the patient with low back pain has vertebral body endplate bone edema (Modic change).

11. The composition for use of claim 10 wherein the patient has Modic change Type I, Modic change Type II, or Modic change Type I+II.

## Patentansprüche

1. Injizierbare Zusammensetzung, umfassend Linezolid Form II und einen Poloxamer 407 und Iohexol umfassenden thermosensitiven Hydrogel-Abgabeträger, zur Verwendung in einem Verfahren zur Behandlung von Schmerzen im unteren Rückenbereich, wobei das Verfahren das Verabreichen einer therapeutisch wirksamen Menge an Linezolid Form II an einen Patienten umfasst, wobei
die Zusammensetzung etwa 2,5 Gew.-% bis 20 Gew.-% Linezolid-Form II, etwa 17 Gew.-% bis 30 Gew.-% Iohexol und etwa 10,8 Gew.-% bis 12,8 Gew.-% Poloxamer 407, bezogen auf das Gewicht der Zusammensetzung, umfasst,
die Zusammensetzung an eine oder mehrere Bandscheiben verabreicht wird;
der Patient zwei Dosen der Zusammensetzung in jede der einen oder mehreren Bandscheiben erhält,
die erste und die zweite Dosis jeweils etwa 150 mg Linezolid Form II enthalten und
das Verabreichungsintervall zwischen der ersten Dosis und der zweiten Dosis etwa 72 Stunden bis etwa 132 Stunden beträgt.

2. Zusammensetzung zur Verwendung nach Anspruch 1, wobei Linezolid Form II in einem aus Poloxamer 407 und Iohexol zusammengesetzten thermosensitiven Hydrogel-Abgabeträger formuliert ist und wobei Linezolid Form II als sterilisiertes mikronisiertes Pulver in einer Dosiseinheit vorgefertigt und vor der Verabreichung in den thermosensitiven Hydrogel-Abgabeträger auf Poloxamer 407-Basis eingebracht wird, wobei der thermosensitive Hydrogel-Abgabeträger als sterilisierte Lösung vorgefertigt ist.

3. Zusammensetzung zur Verwendung nach Anspruch 2, wobei Linezolid Form II in einer Dosiseinheit von etwa 250 mg vorgefertigt und vor der Verabreichung in etwa 5 ml des thermosensitiven Hydrogel-Abgabeträgers eingebracht wird.

4. Zusammensetzung zur Verwendung nach Anspruch 2 oder Anspruch 3, wobei die Zusammensetzung unter Verwendung einer Einführnadel und einer Verabreichungsnadel an die eine oder die mehreren Bandscheiben abgegeben wird.

5. Zusammensetzung zur Verwendung nach Anspruch 2 oder Anspruch 3, wobei die Zusammensetzung unter Verwendung einer Verabreichungsnadel an die eine oder die mehreren Bandscheiben abgegeben wird.

6. Zusammensetzung zur Verwendung nach Anspruch 4 oder Anspruch 5, wobei die Verabreichungsnadel mit einer Luer-Lock-Spritze verbunden ist.

7. Zusammensetzung zur Verwendung nach Anspruch 6, wobei die Verabreichungsnadel und die Luer-Lock-Spritze über ein flexibles Verbindungsstück verbunden sind.

8. Zusammensetzung zur Verwendung nach einem der Ansprüche 1 bis 7, wobei das Verabreichungsintervall zwischen der ersten Dosis und der zweiten Dosis etwa 72 Stunden bis etwa 120 Stunden beträgt.

9. Zusammensetzung zur Verwendung nach Anspruch 8, wobei das Verabreichungsintervall zwischen der ersten Dosis und der zweiten Dosis 72 Stunden, 96 Stunden oder 120 Stunden beträgt.

10. Zusammensetzung zur Verwendung nach einem der vorhergehenden Ansprüche, wobei der Patient mit Schmerzen im unteren Rückenbereich ein Knochenödem der Wirbelkörper-Endplatte (Modic-Veränderung) aufweist.

11. Zusammensetzung zur Verwendung nach Anspruch 10, wobei der Patient Modic-Veränderung Typ I, Modic-Veränderung Typ II oder Modic-Veränderung Typ I+II aufweist

## Revendications

1. Composition injectable comprenant la forme II de linézolide et un support d'administration d'hydrogel thermosensible comprenant du poloxamère 407 et de l'iohexol, destinée à être utilisée dans un procédé de traitement de la lombalgie, le procédé comprenant l'administration d'une quantité thérapeutiquement efficace de la forme II de linézolide à un patient ; dans laquelle
la composition comprend environ 2,5 % à 20 % de forme II de linézolide, environ 17 % à 30 % d'iohexol et environ 10,8 % à 12,8 % de poloxamère 407 par poids de la composition ;
la composition est administrée à un ou plusieurs disques intervertébraux ;
le patient reçoit deux doses de la composition dans chacun du ou des disques intervertébraux ;
les première et deuxième doses contiennent environ 150 mg de forme II de linézolide pour chaque dose ; et
l'intervalle d'administration entre la première dose et la deuxième dose est d'environ 72 heures à environ 132 heures.

2. Composition pour utilisation selon la revendication 1, dans laquelle la forme II de linézolide est formulée dans un support d'administration d'hydrogel thermosensible composé de poloxamère 407 et d'iohexol ; et dans laquelle la forme II de linézolide est prépréparée sous forme de poudre micronisée stérilisée en une unité de dose et chargée sur le support d'administration d'hydrogel thermosensible à base de poloxamère 407 avant l'administration, dans laquelle le support d'administration d'hydrogel thermosensible est prépréparé sous forme d'une solution stérilisée.

3. Composition pour utilisation selon la revendication 2, dans laquelle la forme II de linézolide est prépréparée en une unité de dose d'environ 250 mg et chargée dans environ 5 ml du support d'administration d'hydrogel thermosensible avant l'administration.

4. Composition pour utilisation selon la revendication 2 ou la revendication 3, dans laquelle la composition est administrée au(x) disque(s) intervertébral/intervertébraux en utilisant une aiguille d'introduction et une aiguille d'administration.

5. Composition pour utilisation selon la revendication 2 ou la revendication 3, dans laquelle la composition est administrée au(x) disque(s) intervertébral/intervertébraux en utilisant une aiguille d'administration.

6. Composition pour utilisation selon la revendication 4 ou la revendication 5, dans laquelle l'aiguille d'administration est reliée à une seringue luer-lock.

7. Composition destinée à être utilisée selon la revendication 6, dans laquelle l'aiguille d'administration et la seringue luer-lock sont reliées par l'intermédiaire d'un connecteur flexible.

8. Composition pour utilisation selon l'une quelconque des revendications 1 à 7, dans laquelle l'intervalle de dosage entre la première dose et la deuxième dose est d'environ 72 heures à environ 120 heures.

9. Composition pour utilisation selon la revendication 8, dans laquelle l'intervalle de dosage entre la première dose et la deuxième dose est de 72 heures, 96 heures ou 120 heures.

10. Composition destinée à être utilisée selon l'une quelconque des revendications précédentes, dans laquelle le patient souffrant de lombalgie a un œdème osseux de plaque terminale du corps vertébral (changement modique).

11. Composition destinée à être utilisée selon la revendication 10, dans laquelle le patient a un changement modique de type I, un changement modique de type II ou un changement modique de type I+II.
